# EUROPEAN PATENT APPLICATION

(11) **EP 0 797 996 A1**
(43) Date of publication of application: **01.10.1997**
(21) Application number: 96105068.9
(22) Date of filing: 29.03.1996
(51) Int. Cl.: A61K 35/78

(54) **A pharmaceutical or veterinary composition which contains at least one volatile oil, at least one alcohol and at least one fixed oil**

(71) Applicant: Ornaquin Ltd., 64047 Tel Aviv (IL)
(72) Inventor: Orna, Levin, 40593 (IL); David, Marcos, 40230 (IL)
(74) Representative: Beetz & Partner Patentanwälte

(57) **Abstract**

A pharmaceutical or veterinary composition which includes one or more volatile oil, one or more alcohol and one or more fixed oil and/or one or more emollient ester of fatty acid derived from vegetable oils.

## Description

### FIELD AND BACKGROUND OF THE INVENTION

The present invention relates to a pharmaceutical or veterinary composition.

Volatile oils have for centuries been used in various ways to treat medical problems. Their use is found in folk medicine, natural therapy, aromatherapy and even in traditional (Western) medicine.

Volatile oils in connection with the present invention are those essential oils embraced by the definition in Hackh's Chemical Dictionary, 4th Edition, page 248.

A number of volatile oils found in plants are known to repel or kill insects. The volatile oils mights be part of the mechanisms which plants use to protect themselves from attack by insects and animals.

This property to kill or repel insects has also been used in medicine. For example, the use of volatile oils in the treatment of parasitic infestations is described in "The Manual of Natural Therapy" by M. Olshevsky, B. Noy and M. Zwang (Published by Facts of Life, New York, 1989). For scabies, the following treatment is suggested (pp 183, 185): "Use the essence of geranium 5% in olive oil base and massage the affected part of the skin once per day until condition improves." For lice, the following is suggested (p.185): "Rub the whole body with the following combination in olive oil base: crushed garlic 10%, lavender essence 3%, thyme 2%, rosemary 4%. Do this treatment once per day until condition improves." Another treatment for lice suggests (p. 186) using Aniseed oil or a 1:1 mixture of Sassafras oil and quassis oil.

Lice belong to the group of external parasites living on warm-blooded animals. In humans, lice are responsible for pediculosis, a parasitic infestation of the skin of the scalp, trunk or pubic areas. There are three different varieties: (1) Pediculosis pubis caused by Phthirus pubis; (2) Pediculosis corporis, caused by Pediculus humanus humanus; (3) Pediculosis capitis, caused by Pediculus humanis capitis.

In animals lice mainly affect birds.

Scabies is a common dermatitis caused by infestation with the mite Sarcoptes scabisi. It affects humans and is also found in various forms in animals such as dogs, cattle, sheep, camels and birds.

Both scabies and pediculosis are conditions which affect millions of humans world-wide. A variety of products and treatments have been tried over the years to treat scabies or lice infestations but none has succeeded in eradicating these two conditions. Epidemics of scabies and of pediculosis appear to be cyclical in nature.

The volatile oils are rather expensive. Moreover, the traditional way to use pure volatile oils is usually hampered by the tendency of these oils to often cause a burning sensation and erythema when applied to the skin. Diluting the volatile oils in a fixed oil such as olive oil may reduce these side-effects but also reduces their potency. Treatment is less effective and often requires repeated applications. (Fixed oils in connection with the present invention are those embraced by the definition in Hackh's Dictionary (Chemical). 4th Edition, page 269).

Dissolving a volatile oil in an alcohol, e.g. ethanol, retained the anti-insect properties but also retained the undesired side-effects, e.g., the burning sensation on the skin and erythema.

### SUMMARY OF THE INVENTION:

The pediculicidal activity of various formations was tested in the laboratory on human body louse pediculus humanus humanus according to the following methods:

Body lice were reared in the laboratory by feeding them every second day on rabbits. Lice were placed on the shaved abdomen of a white rabbit and left until they fed to satisfy. Outside the host the lice were maintained at a temperature of 30 + 1 degree C and relative humidity of 70+10%.

For each test 50 lice (10 males, 10 females and 30 nymphs) were placed on a 7 cm white filter paper disc (Whatman No. 2) and exposed to 1 g of the test formulation. The lice were left in contact with the formulation for 15 minutes. Thereafter they were removed and shampooed for one minute with a regular shampoo and then washed for one minute under running tap water. After treatment the lice were transferred to a fresh filter paper disc and incubated overnight at optimum temperatures and humidities. Mortality was determined after 24 hours.

### DESCRIPTION OF THE PREFERRED EMBODIMENTS

In order to examine the ovicidal activity lice were allowed to oviposit on human hair. Fifty 2-6 day old eggs were tested according to the same procedure as for lice. Mortality count was made after 10 days. The testing for lice and eggs was repeated 3 times. As a control 40% ethyl alcohol was used.

Pure Rosemary Oil and Anise Oil were tested for their efficacy in killing lice. The oils were also diluted in Olive Oil or in Ethanol (95%) and efficacy in killing lice again tested. The results are shown in Table 1.

**Table 1**

| **Example Number** | **Percentage (v/v) Volatile Oil** | | **Diluent (to 100% v/v)** | **Percentage Lice Killed** | **Percentage Eggs Killed** |
|---|---|---|---|---|---|
| 1 | Rosemary Oil | 100% | - | 100% | 28.8% |
| 2 | Anise Oil | 100% | - | 100% | 79.7% |
| 3 | Rosemary Oil | 33% | Olive Oil | 34% | - |
| 4 | Anise Oil | 33% | Olive Oil | 68% | - |
| 5 | Rosemary Oil | 33% | Ethanol (95%) | 100% | 42% |
| 6 | Anise Oil | 33% | Ethanol (95%) | 100% | 72% |
| 7 | Rosemary Oil | 33% | Olive Oil | 100% | 18% |
| | Anise Oil | 33% | | | |
| 8 | Rosemary Oil | 28% | Ethanol (95%) | 100% | 61.1% |
| | Anise Oil | 5% | | | |
| 9 | Rosemary Oil | 5% | Ethanol (95%) | 100% | 88.9% |
| | Anise Oil | 28% | | | |
| 10 | Rosemary Oil | 16.5% | Ethanol (95%) | 100% | 76.3% |
| | Anise Oil | 16.5% | | | |

It has therefore been desirable to find a pharmaceutical or veterinary composition which has the desired anti-insect properties, in which a lower concentration of volatile oils may be used and which has no adverse effect on the skin.

The present invention thus consists in a pharmaceutical or veterinary composition comprising:
a) one or more volatile oils (as herein defined);
b) one or more alcohol; and
c) one or more fixed oil (as herein defined) and/or one or more emollient ester of fatty acid derived from the vegetable oils.

The composition according to the present invention is preferably a solution.

Suitable volatile oils are, e.g., anise oil, calendula oil, quassia oil, rosemary oil and Sassafras oil.

Suitable alcohols are, e.g., ethanol and isopropyl alcohol.

Suitable fixed oils are, e.g., almond oil, avocado oil, maize oil, olive oil, peanut oil, soya oil, sunflower oil, sesame seed oil and Safflower oil.

Suitable esters (of the kind defined above) are, e.g., medium chain triglycerides (MCT), caprylic/capric triglyceride; isopropyl myristate; propylene glycol dicaprylate - dicaprate; and isopropyl palmitate. MCT are e.g. those as defined in German Pharmacopea, 8th Edition.

The amounts of each ingredient present in the composition according to the present invention may vary according to the specific ingredient utilized. said composition suitably comprise:
a) 0.5-50%, preferably 5-30% of volatile oil;
b) 10-60%, preferably 20-40% of alcohol; and
c) 10-85%, preferably 20-60% of a fixed oil and/or of the ester. (All percentages and v/v).

Some compositions according to the present invention were prepared by admixing the various ingredients. The anti-lice activity of a number of said compositions were tested in the same manner as previously described. The results are shown in Table 2.

**Table 2**

| **Example Number** | **Percentage (v/v) Volatile Oil** | | **Diluent (% v/v)** | | **Percentage Lice Killed** | **Percentage Eggs Killed** |
|---|---|---|---|---|---|---|
| 12 | Rosemary Oil | 15% | Olive Oil | 20% | 100% | 17.2% |
| | Anise Oil | 15% | *MCT | 20% | | |
| | | | Isopropanol | 30% | | |
| 13 | Rosemary Oil | 15% | Olive Oil | 35% | 32% | - |
| | Anise Oil | 15% | MCT | 35% | | |
| 14 | Rosemary Oil | 5% | Olive Oil | 25% | 99% | 21% |
| | Anise Oil | 15% | MCT | 25% | | |
| | | | Isopropanol | 30% | | |
| 15 | Rosemary Oil | 5% | Olive Oil | 40% | 15% | - |
| | Anise Oil | 15% | MCT | 40% | | |
| 16 | Rosemary Oil | 5% | Olive Oil | 30% | 89% | - |
| | Anise Oil | 5% | MCT | 30% | | |
| | | | Isopropanol | 30% | | |
| 17 | Rosemary Oil | 15% | Olive Oil | 25% | 94% | - |
| | Anise Oil | 5% | MCT | 25% | | |
| | | | Isopropanol | 30% | | |
| 18 | Rosemary Oil | 10% | Olive Oil | 25% | 94% | - |
| | Anise Oil | 10% | MCT | 25% | | |
| | | | Isopropanol | 30% | | |
| 19 | Rosemary Oil | 5% | Olive Oil | 10% | 97.3% | 40.6% |
| | Anise Oil | 15% | Ethanol (95%) | 35% | | |
| | | | MCT | 30% | | |
| | | | Isopropanol | 5% | | |
| 20 | Rosemary Oil | 5% | MCT | 40% | 93% | 56.8% |
| | Anise Oil | 15% | Isopropanol | 40% | | |
| 21 | Rosemary Oil | 5% | MCT | 40% | 90% | 41.2% |
| | Anise Oil | 15% | Ethanol (95%) | 40% | | |
| 22 | Rosemary Oil | 5% | MCT | 55% | 98.7% | 58.9% |
| | | | Isopropanol | 40% | | |
| 23 | Anise Oil | 15% | MCT | 45% | 100% | 59.6% |
| | | | Isopropanol | 40% | | |

| | | | | | | |
|---|---|---|---|---|---|---|
| S* MCT = Medium Chain Triglycerides | | | | | | |

The MCT utilized was Estasan^{R} manufactured by OS Industries APS, Copenhagen. Some of these preparations were tried on human volunteers and they did not cause the burning sensation or reddening of the skin associated with high concentration of volatile oils.

## Claims

1. A pharmaceutical or veterinary composition comprising:
a) one or more volatile oils (as herein defined);
b) one or more alcohols and
c) one or more fixed oils (as herein defined) and/or one or more emollient esters of fatty acids derived from vegetable oils.

2. A composition according to claim 1, wherein the one or more volatile oils are selected among anise oil, calendula oil, quassia oil, rosemary oil and Sassafras oil.

3. A composition according to claim 1 or 2, wherein the alcohol is ethanol or isopropyl alcohol.

4. A composition according to one or several of the preceding claims, wherein the one or more fixed oils are selected among, almond oil, avocado oil, maize oil, olive oil, peanut oil, soya oil, sunflower oil, sesame seed oil and Safflower oil.

5. A composition according to one or several of the preceding claims, wherein the one or more esters (of the kind defined in claim 1) are selected among medium chain triglycerides (MCT), caprylic/ capric triglycerides; isopropyl myristate; propylene glycol dicaprylate; propylene glycol dicaprate and isopropyl palmitate.

6. A composition according to one or several of the preceding claims, which comprises 0.5 - 50 %, preferably 5 - 30 %, of one or more volatile oils.

7. A composition according to one or several of the preceding claims, which comprises 10 - 60 %, preferably 20 - 40 %, of one or more alcohols.

8. A composition according to one or several of the preceding claims, which comprises 10 - 85 %, preferably 20 - 60 %, of one or more fixed oils and/or one or more of said esters.

9. A composition according to one of the preceding claims, which is a solution.

10. A composition according to one of the preceding claims for the treatment or prevention of scabies or lice infestations in humans or animals.
